(19) Europäisches Patentamt / European Patent Office / Office européen des brevets

(11) EP 3 522 961 B1

(12) **EUROPÄISCHE PATENTSCHRIFT**

(45) Veröffentlichungstag und Bekanntmachung des Hinweises auf die Patenterteilung:
**11.05.2022 Patentblatt 2022/19**

(21) Anmeldenummer: **17807690.7**

(22) Anmeldetag: **05.10.2017**

(51) Internationale Patentklassifikation (IPC):
***A61M 3/02*** *(2006.01)* ***A61M 13/00*** *(2006.01)*
***A61B 1/015*** *(2006.01)*

(52) Gemeinsame Patentklassifikation (CPC):
**A61M 13/003; A61B 1/015; A61M 3/0216;**
A61M 2205/3344; A61M 2205/3365; A61M 2205/50;
A61M 2205/52

(86) Internationale Anmeldenummer:
**PCT/DE2017/000332**

(87) Internationale Veröffentlichungsnummer:
**WO 2018/064996 (12.04.2018 Gazette 2018/15)**

(54) **VORRICHTUNG ZUR INTRAOPERATIVEN BESTIMMUNG DER WIDERSTANDSBEIWERTE VON VERSCHIEDENEN MEDIZINISCHEN INSTRUMENTEN BEI DER VERWENDUNG EINER MEDIZINTECHNISCHEN FLUIDPUMPE**

DEVICE FOR INTRAOPERATIVE DETERMINATION OF DRAG COEFFICIENT VALUES OF DIFFERENT MEDICAL INSTRUMENTS IN THE USE OF A MEDICAL FLUID PUMP

DISPOSITIF POUR LA DÉTERMINATION PEROPÉRATOIRE DES COEFFICIENTS DE RÉSISTANCE DE DIFFÉRENTS INSTRUMENTS MÉDICAUX LORS DE L'UTILISATION D'UNE POMPE D'IRRIGATION MÉDICALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.10.2016 DE 102016011819**

(43) Veröffentlichungstag der Anmeldung:
**14.08.2019 Patentblatt 2019/33**

(73) Patentinhaber: **W.O.M. World of Medicine GmbH**
**10587 Berlin (DE)**

(72) Erfinder:
- **ZEYSSIG, Andreas**
  **03238 Finsterwalde (DE)**
- **SCHULZE, Stephan**
  **12101 Berlin (DE)**
- **ILIK, Ibrahim**
  **10551 Berlin (DE)**

(74) Vertreter: **Jungblut & Seuss**
**Patentanwälte**
**Wittestraße 30J**
**13509 Berlin (DE)**

(56) Entgegenhaltungen:
**US-A1- 2007 078 370 US-A1- 2013 267 779**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Gegenstand der vorliegenden Erfindung ist ein Verfahren zur Bestimmung der Widerstandsbeiwerte insbesondere von verschiedenen Schaft- und Endoskop-Kombinationen beider Verwendung einer medizintechnischen Fluidpumpe, z.B. im Rahmen der Arthroskopie.

Bei verschiedenen medizinischen Eingriffen in das Körperinnere werden Fluide, z.B. Gase oder Flüssigkeiten in das Körperinnere ein- und ausgeführt. Beispielhaft genannt sei hier die Arthroskopie, bei der beispielsweise im Rahmen einer Kniegelenksuntersuchung oder einer therapeutischen Behandlung das Knie mit einer Spülflüssigkeit gespült wird. Eine andere beispielhafte Behandlung ist die Laparoskopie, in der während eines therapeutischen Eingriffs Gase (z.B. $CO_2$) in das Körperinnere geführt werden. Im Rahmen dieser Prozeduren ist die Messung, die Regelung und vor allem die Begrenzung des Druckes im Körperinneren von besonderer Bedeutung. Bei therapeutischen Eingriffen ist es insbesondere notwendig, einen gewissen Fluidfluss zu gewährleisten, um beispielsweise Rauch oder Blut aus dem Körperinneren auszuspülen, gleichzeitig aber den Druck zu begrenzen, um das Körpergewebe nicht zu beschädigen. Hierzu stehen verschiedene Vorrichtungen und Verfahren zur Verfügung.

Zur Vermeidung verschiedenster Nachteile älterer Verfahren wurde in jüngerer Zeit ein Verfahren und eine darauf angepasste Vorrichtung vorgestellt, die den Körperinnendruck während des Pumpenbetriebes besonders präzise bestimmt (WO 2015/144120), ohne einen Drucksensor in der Körperhöhle zu benötigen. Bei diesem Verfahren werden die Daten eines Drucksensors, welcher sich außerhalb der betreffenden Körperhöhle befindet, als Grundlage für eine Schätzung des Druckes im Körperinneren herangezogen. Für die Schätzung des Körperinnendrucks wird ein mathematisches Modell herangezogen, welcher das medizintechnische Gesamtsystem, bestehend z.B. aus Druckregler, regelbarem Pumpenmotor, Zuführleitung, Drucksensor, medizinische Zuführvorrichtung (z.B. Schaft mit Endoskop), Körperhöhle und ggf. Fluidauslass (z.B. Absaugvorrichtung) durch einen Satz von Differenzialgleichungen beschreibt und in einem sogenannten Zustandsraummodel zusammenfasst. Die Einzelheiten sind in der WO 2015/144120 beschrieben.

Aus dem Stand der Technik sind auch folgende Dokumente US 2007/0078370 A1 und US 2013/0267779 A1 bekannt. Diese Dokumente offenbaren medizinische Vorrichtungen zum Einbringen von Fluiden in Körperhöhlen sowie Verfahren zur Auswahl instrumentenspezifischer Strömungswiderstandskoeffizienten.

Wie sich beim Betrieb eines derartigen Systems herausgestellt hat, sind viele der schätzungsentscheidenden Parameter der oben beschriebenen Einzelkomponenten im Wesentlichen konstant. Es hat sich jedoch herausgestellt, dass die verschiedenen medizinischen Zuführvorrichtungen (z.B. die verschiedenen möglichen Schafte) sehr unterschiedliche Parameter, insbesondere Strömungsparameter, aufweisen. Je nach verwendeter Kombination von Schaft- und Endoskop (nachfolgend auch: Instrument) tritt ein sehr unterschiedlicher Druckabfall auf.

Für den Betrieb der medizintechnischen Flüssigkeitspumpe muss daher vor Beginn einer Operation für jedes Instrument der jeweilige Widerstandsbeiwert (siehe unten) gemessen werden. Dies kann beispielsweise so erfolgen, dass im Rahmen einer "Open-Flow-Messung" ein Flüssigkeitsfluss erzeugt wird und der Druckabfall gegenüber dem Umgebungsdruck gemessen wird. Die Messung erfolgt dabei naturgemäß außerhalb des Gelenks. Der sich einstellende Strömungsdruck entspricht dem Instrumentendruck, d.h. dem Widerstandsbeiwert der zugrundeliegenden Kombination von Schaft- und Endoskop. Die Nachteile dieser Messmethodik liegen auf der Hand: Der wichtigste Nachteil dieser Messmethode ist, dass bei jedem Instrumentenwechsel- auch im Falle eines intraoperativen Instrumentenwechsels - eine derartige Messung durchgeführt werden muss. Nachteilig hieran ist besonders der Zeitbedarf, der mindestens 15 bis 30 Sekunden beträgt. Weiterhin nachteilig ist, dass für die Messung eine gewisse Menge Fluid verwendet werden muss, welches nicht weiter zum Einsatz kommen kann. Dieser Zeit- und Fluidbedarf wird von den Ärzten, die derartige Systeme nutzen, nur schwer akzeptiert.

Wird der Druck entlang des Flüssigkeitsstroms zwischen zwei spezifischen Systempunkten betrachtet, ergibt sich bei gleichen Strömungsgeschwindigkeiten und konstanter Dichte der in Gleichung 1 gezeigte Zusammenhang:

**Gleichung 1:** $$\Delta p = p_1 - p_2$$

Beispielsweise beschreibt $\Delta p$ den Druckabfall über die verwendete Schaft- und Endoskop Kombination (der sogenannte Instrumentendruck) aus der Differenz vom Strömungsdruck im Schlauch und dem Ruhedruck im Gelenk. Der Ruhedruck im Gelenk ist die Regelgröße der Pumpe und wird aus den oben geschilderten Gründen nicht gemessen. Um den Druck im Gelenk zu bestimmen muss neben den messbaren Strömungsdruck der Instrumentendruck gemessen werden. Hierzu kann eine Kennlinie bestimmt werden, die auf den dimensionslosen Widerstandsbeiwerten $\zeta_1$ und $\zeta_2$ gemäß Gleichung 2 basiert:

**Gleichung 2** $$\Delta p = \varsigma_1 \cdot {n_1}^2 + \varsigma_2 \cdot n_1$$

Durch Einsetzen von Gleichung 2 in Gleichung 1 und Umstellung nach $p_2$ ergibt sich folgende statische Messgleichung 3:

**Gleichung 3** $$\hat{p}_2 = p_1 - (\varsigma_1 \cdot n_1^{\ 2} + \varsigma_2 \cdot n_1)$$

Hierbei stellt die linke Seite der Gleichung ($\hat{p}_2$) eine Schätzung des Gelenkdrucks dar. Um die Widerstandsbeiwerte der Gleichung 2 zu bestimmen, müssen mindestens drei Wertepaare ($\Delta$p) bei drei unterschiedlichen Flüssen ($n_1$) aufgenommen werden. Eine derartige Messung ist in der Figur 1 abgebildet. Hierbei werden drei verschiedene Flüsse eingestellt und der jeweilige Differenzdruck gemessen. Wie in Figur 1 (oben) dargestellt stellt sich nach einer bestimmten Zeit ein stationärer Endwert ein. Die Einstellung des Flusses erfolgt durch Regelung der Motordrehzahl der Pumpe. Die Bestimmung des Druckes erfolgt in Open-Flow-Modus, d.h. gegenüber dem Umgebungsdruck.

Die oben genannte Methodik enthält einige Nachteile:

1) Um möglichst genaue Widerstandsbeiwerte zu erhalten, muss jeweils abgewartet werden, bis das Signal den stationären Endwert erreicht hat.

2) Für die Identifikation der Widerstandbeiwerte müssen mindestens drei Drehzahlstufen angefahren werden, anders ist das zugrunde gelegte Gleichungssystem nicht lösbar.

3) Aufgrund des Zeitbedarfs für die Einstellung des stationären Endwertes in Kombination mit den benötigten Drehzahlstufen ergibt sich eine für die Anwendung verbesserungsfähige Dauer der Instrumentenerkennung außerhalb des Gelenks.

4) Das Vorgehen ist ungeeignet, um eine Identifikation des Instrumentes (d.h. die Bestimmung des Instrumentendrucks) im Gelenk durchzuführen. Hierbei würde sich ein zu hoher Überdruck im Gelenk ergeben.

5) Das für die Messung benötigt Fluid wird für die operative Maßnahme nicht genutzt.

Die Erfindung ist in angehängten Ansprüchen definiert. Die abhängigen Ansprüche definieren bevorzugte Ausführungsformen der Erfindung.

Aufgabe der vorliegenden Erfindung ist es daher, die Messung der Widerstandsbeiwerte für die verschiedenen Instrumente zu vereinfachen. Die Messung soll dabei schneller und grundsätzlich im Körper (z.B. im Gelenk) erfolgen und möglichst wenig Fluid oder Gas verbrauchen.

Die Lösung der Aufgabe erfolgt durch die Vorrichtung gemäß Anspruch 1.

Die erfindungsgemäße Vorrichtung ermittelt die Widerstandsbeiwerte $\zeta_1$ und $\zeta_2$ bereits bei einem einmaligen Anfahren der Pumpe mit akzeptabler Genauigkeit. Eine höhere Genauigkeit wird erzielt, wenn das Anfahren mehrfach durchgeführt wird. Als optimal hat sich das zweifache Anfahren der Pumpe herausgestellt.

Der Begriff "Anfahren der Pumpe" umfasst insbesondere die Veränderung der Pumpleistung von 0 ml/min auf eine für das gewünschte Instrument und die Verwendung angepasste Pumpleistung (z.B. 25 l/min für die Insufflation oder 500 ml/min für die Arthroskopie), z.B. durch Einschalten einer peristaltischen Rollenradpumpe unter Einstellung einer Solldrehzahl. In besonderen Fällen kann die Messung auch in einer Weise durchgeführt werden, dass die Pumpe von einer kleinen Leistung auf eine deutlich größere Leistung umgestellt wird (z.B. von 2,5 l/min auf 25 l/min für die Insufflation oder von 50 ml/min auf 500 ml/min für die Arthroskopie). Auch eine derartige Durchführung wird soll vom Begriff "Anfahren der Pumpe" umfasst sein. Eine derartige, weniger bevorzugte Ausführungsform erfordert die Anpassung der weiter unten dargestellten Berechnungen, insbesondere der Berechnung des Druckverlustterms $\Delta$p.

Die Lösung der oben gestellten Aufgabe erfolgt durch eine medizintechnische Vorrichtung zum Einbringen von Fluiden in Köperhöhlen, enthaltend eine regelbare Fluidpumpe, eine Speichereinheit, eine Zuführleitung, einen Drucksensor in der Zuführleitung, ein an die Zuführleitung anschließbares medizinisches Instrument dadurch gekennzeichnet, dass der durch den Drucksensor gemessene Druck eine Eingangsvariable eines mathematischen Schätzsystems ist, welches mathematisch einen Zustandsraum beschreibt, welcher den tatsächlichen Druck in der Körperhöhle abschätzt und mittels dieses Schätzwertes die Leistung der Pumpe regelt, wobei die zur Schätzung des Druckes nötigen Widerstandsbeiwerte $\zeta_1$ und $\zeta_2$ des medizinischen Instrumentes dadurch bestimmt werden, dass für beim Anfahren der Pumpe für eine bestimmte Zeit der Druckverlauf ausgewertet, hieraus eine Kennlinie ermittelt und die Kennlinie in der Speichereinheit der Pumpe abgespeichert wird. Um die eingangs genannten Nachteile der Open-Flow-Methode zu kompensieren wird daher folgende Messmethode vorgeschlagen:

Für eine gegebene medizintechnische Flüssigkeitspumpe wird in einer ersten Versuchsreihe eine Kennlinienschar aufgenommen. Hierzu werden die für den Betrieb der Flüssigkeitspumpe vorgesehenen Instrumente, (d.h. Schaft- und Endoskop Kombinationen) mit der Pumpe gekoppelt und der jeweilige flussabhängige Instrumentendruck gemessen und ausgewertet. Die für ein bestimmtes Instrument gemessenen Werte können als Kennlinie dargestellt werden. Eine Kennlinienschar, die derartige Kennlinien verschiedener Instrumente zeigt, ist beispielhaft in vereinfachter Form in der Figur 2 dargestellt. Dabei zeigt sich, dass die unterschiedlichen Widerstandscharakteristiken der Instrumente vor allem vom effektiven Strömungsquerschnitt abhängen. Man kann davon ausgehen, dass sich andere physikalische Abhängigkeiten zeitinvariant verhalten.

Im Ergebnis zeigen sich unterschiedliche, vom Strömungsquerschnitt abhängige Widerstandsbeiwerte ($\zeta_{1,z}$ und $\zeta_{2,z}$), wobei z die Anzahl der aufgenommenen Kennlinien angibt. Aus diesen Kennlinien wird als a priori-Wissen die $\zeta_2$Werte in einer Speichereinheit der Pumpe hinterlegt.

Für die Messung und Speicherung der Kennlinien kommen unterschiedliche Wege in Betracht. Es ist möglich, dass bei der Herstellung der Pumpe sämtliche hierfür zugelassenen Instrumente gemessen und die Widerstandsbeiwerte bzw. Kennlinien gespeichert werden. In einer anderen Ausführungsform wird vor jeder Anwendung, d.h. nach dem Anschluss des jeweiligen Instrumentes an die Pumpe eine Messung und Speicherung der Widerstandsbeiwerte bzw. Kennlinien vorgenommen. Möglich ist natürlich auch, dass die Pumpe mit einigen gespeicherten Kennlinien vermarktet wird, der Anwender aber auch zusätzlich für seine bevorzugten Instrumente die Widerstandsbeiwerte bzw. Kennlinien individuell vermessen und zusätzlich zu den bereits gespeicherten Daten abspeichern kann.

In jedem Fall kann vor oder während einer Operation der Messvorgang neu gestartet werden, so dass intraoperativ Anpassungen möglich sind.

Um einen Algorithmus für die Instrumentenerkennung im Körper (z.B. im Gelenk) abzuleiten wird das in der Gleichung 2 beschriebene Polynom wie folgt umgeschrieben:

**Gleichung 4**

$$\varsigma_1 = \frac{\Delta p - \varsigma_2 \cdot n_1}{n_1^{\,2}} = \frac{(p_1 - p_2) - \varsigma_2 \cdot n_1}{n_1^{\,2}}$$

Gleichung 4 beschreibt den Strömungswiderstand $\zeta_1$als Funktion der messbaren Drehzahl, des messbaren Strömungsdruckes $p_1$, dem nicht messbaren Ruhedruck $p_2$ im Körper sowie einem vorher festgelegten Wert für den Strömungswiderstand $\zeta_2$. Der Strömungswiderstand $\zeta_2$ wird innerhalb bestimmter Drehzahlbereiche als konstant angenommen. Durch eine kurze, konstante, Drehzahlförderung ergibt sich anhand des Druckanstieges ein geeigneter $\zeta_2$ Wert, der aus dem Speicher selektiert wird.

Um $\zeta_1$ mittels der Gleichung 4 berechnen zu können, muss der Verlustterm $\Delta$p bestimmt werden. Dies wird anhand der Figur 3 beschrieben:

Für die Berechnung des Druckverlustterms $\Delta$p gelten folgende Bedingungen:

- $p_1 = p_2$ für $n_1 = 0$ im Zeitraum $(t_1\text{-}t_0)$ und $t_3 > 0$
- $p_1 = \Delta p + p_2$ für $n_1 > 0$

Unter Berücksichtigung der angegebenen Bedingungen kann zum Zeitpunkt $t_2$ der messbare Strömungsdruck ermittelt werden. Die Ermittlung des Ruhedrucks $p_2$ erfolgt für Zeitpunkte $t >= t_3$, nachdem die Dynamik des Messsignals abgeklungen ist. Der Verlustterm ergibt sich aus der Differenz $(p_1 - p_2)$.

Zu beachten ist, dass die Genauigkeit der Berechnung von $\Delta$p von der Größe einer eventuellen Leckage abhängt. Im Falle, dass die bestimmten Instrumentenparameter außerhalb eines plausiblen Bereichs liegen, wird eine im Speicher hinterlegte Kennlinie gewählt.

Ein Vergleich des hier offenbarten Verfahrens mit dem oben geschilderten Verfahren gemäß Stand der Technik zeigt die überraschenden Vorteile der vorliegenden Erfindung:

- Das bisherige Verfahren (Open-Flow-Methode) benötigt zur Bestimmung der Widerstandsbeiwerte $\zeta_1$ und $\zeta_2$, die Einstellung von drei verschiedenen Flüssen bei der Pumpe. Im Vergleich benötigt das erfindungsgemäße Verfahren nur das ein- oder zweimalige Anfahren der Pumpe.
- Das Identifikationsverfahren gemäß Stand der Technik dauert 15 bis 30 Sekunden, während das erfindungsgemäße Verfahren (bei zweimaligem Anfahren der Pumpe) lediglich ca. 7 Sekunden benötigt.
- Das Identifikationsverfahren gemäß Stand der Technik muss außerhalb des Körpers durchgeführt werden. Das erfindungsgemäße Identifikationsverfahren wird standardgemäß innerhalb des Körpers durchgeführt, kann jedoch auch außerhalb des Körpers durchgeführt werden.
- Das Identifikationsverfahren gemäß Stand der Technik benötigt Zeit. Der Anwender muss das Verfahren abwarten, bis er mit dem Eingriff beginnen kann. Das erfindungsgemäße Verfahren läuft während der Anwendung im Hintergrund ab, wodurch der Anwender nicht beeinträchtigt wird.

- Nach dem Identifikationsverfahren gemäß Stand der Technik kann der Operateur nicht unmittelbar mit den Eingriff beginnen. Hierzu muss er zunächst einen gewissen Fluidfluss in das Körperinnere erzeugen (VorAufblähung). Im Rahmen des erfindungsgemäßen Verfahrens wird der zur Identifikation erzeugte Fluid-Fluss standardmäßig bereits zur Voraufblähung der Körperhöhle genutzt. Die Menge ungenutzten Fluides wird somit minimiert.

Insgesamt ergeben sich daher erhebliche Vorteile hinsichtlich der Schnelligkeit und der Anwenderfreundlichkeit. Dabei ist es von besonderer Wichtigkeit, dass die Genauigkeit des Erfindungsverfahrens in etwa der Genauigkeit den aus dem Stand der Technik bekannten Verfahren entspricht. Figur 5 zeigt die Daten einer tatsächlichen Druckmessung in einem Gelenkdummy (schwarz dargestellt) verglichen mit den Schätzdaten eines Systems gemäß WO2015/144120 (grau dargestellt). Die tatsächlichen Werte sind nie größer als die Schätzwerte, in der Regel sind sie geringfügig kleiner als die Schätzdaten, was aus Sicherheitsgründen zu bevorzugen ist. Figur 6 zeigt die Daten einer tatsächlichen Druckmessung in einem Gelenkdummy (schwarz dargestellt) verglichen mit den Schätzdaten eines erfindungsgemäßen Systems (grau dargestellt). Auch hier sind die tatsächlichen Werte nie größer als die Schätzwerte, in der Regel sind sie ebenfalls geringfügig kleiner als die Schätzdaten, was auch hier aus Sicherheitsgründen vorteilhaft ist. Im Ergebnis zeigt sich eine in etwa vergleichbare Genauigkeit der Druckschätzung im Gelenk.

Die vorliegende Erfindung betrifft eine medizinische Fluidpumpe zum Spülen von Körperhöhlen (z.B. Gelenkhöhlen). Dabei kann es sich sowohl um eine Flüssigkeitspumpe, wie auch um einen Insufflator handeln. Eine Flüssigkeitspumpe, die nach Art einer peristaltischen Rollenradpumpe arbeitet ist erfindungsgemäß bevorzugt. Die geregelte Pumpe fördert ein Fluid über einen Schlauch und ein medizinisches Instrument, beispielsweise einem Schaft mit Optik in eine Körperhöhle, beispielsweise ein Kniegelenk. Die Körperhöhle kann dabei eine Vorrichtung zur Flüssigkeitsabfuhr aufweisen. Die Pumpe wird bestimmungsgemäß so betrieben, dass sie einen Überdruck in der Körperhöhle aufbaut, der die Körperhöhle aufweitet (aufbläht). Bei der erfindungsgemäßen Vorrichtung wird der Körperinnendruck, wie eingangs geschildert, im Wege einer Schätzung ermittelt. Ein außerhalb der Körperhöhle befindlicher Drucksensor im oder am Schlauch ermittelt dabei Druckdaten, die den Eingangsparameter für eine Schätzung darstellt. Dieses mathematische Schätzsystem beschreibt einen Zustandsraum, welcher den tatsächlichen Druck in der Körperhöhle abschätzt und Mittels dieses Schätzwertes die Leistung der Pumpe regelt. Eine derartige Vorrichtung ist in der WO 2015/144120 beschrieben. Die erfindungsgemäße Vorrichtung weist über die dort beschriebene Pumpe hinaus einen zusätzlichen Speicher auf, in dem die Ergebnisse der a priori-Wissen niedergelegt sind.

Die Speichereinheit kann in einem unveränderlichen Chip (z.B. einem EPROM) realisiert sein. Alternativ kommen natürlich auch andere, insbesondere austauschbare bzw. veränderbare Speichermedien in Betracht. Es kann dabei vorgesehen sein, dass die Speichereinheit oder die gespeicherten Daten durch Updates veränderbar sind, beispielsweise durch Austausch der Speichereinheit oder durch das Laden neuer Daten über entsprechende Schnittstellen. Das Laden neuer Daten kann optional auch über das Internet erfolgen, wobei natürlich die Sicherheit des Ladevorgangs sichergestellt sein muss, insbesondere hinsichtlich der Authentizität der Datenquelle.

Zur Ermittlung des a priori-Wissen der Kennlinien kann der Pumpenhersteller beispielsweise alle für die Pumpe vorgesehenen Instrumente (d.h. aller Kombinationen von Schaft und Endoskop) vermessen und diese Messdaten in der Speichereinheit jeder Pumpe vor der Auslieferung speichern.

Alternativ und/oder ergänzend können Messdaten erhoben werden, wobei verschiedene Instrumente durch ein Proportionalventil simuliert werden. Dies ist möglich weil, wie oben ausgeführt, die unterschiedlichen Widerstandscharakteristiken der Instrumente vor allem vom effektiven Strömungsquerschnitt abhängen, der durch unterschiedliche Einstellungen eines Proportionalventils simuliert werden kann.

Alternativ und/oder ergänzend können die Widerstandsbeiwerte $\zeta_1$ und $\zeta_2$ von vorgesehenen Instrumenten durch Vermessungen in der Speichereinheit jeder Pumpe hinterlegt werden. Sobald die Pumpe in Betrieb genommen wird, werden die Daten des Drucksensors, d.h. der sich einstellende Druck im Schlauch mit den gespeicherten Kennwerten verglichen. Diejenigen Widerstandsbeiwerte mit der größtmöglichen Übereinstimmung mit den Messdaten werden ausgewählt und die Widerstandsbeiwerte $\zeta_1$ und $\zeta_2$im Rahmen des Schätzsystems zur Schätzung des Körperinnerdrucks verwendet.

Ein möglicher Programmablauf wird in der Figur 4 gezeigt. Die erfindungsgemäße Vorrichtung kann mit verschiedenen Fluidabflusseinrichtungen betrieben werden. Es ist möglich den Abfluss aus die Körperhöhle über einen Öffnung (z.B. einen Schnitt) oder einen Schlauch passiv zu gewährleisten. Möglich ist auch, eine Pumpe vorzusehen, die das Fluid aus der Körperhöhle abpumpt. Bevorzugt sind Pumpsysteme mit zwei peristaltischen Schlauchpumpen (Doppelrollenpumpen), wovon eine Rollenpumpe den Zufluss (Förderpumpe) und die andere den Abfluss (Absaugpumpe) gewährleistet. Das erfindungsgemäße System arbeitet auch mit mehreren Abflusssystemen.

Die erfindungsgemäße Vorrichtung kann insbesondere bei Flüssigkeitspumpen in der Arthroskopie, Urologie, Hysteroskopie, Laparoskopie oder für Wirbelsäulenuntersuchungen Verwendung finden. Weiterhin können mittels der erfindungsgemäße Vorrichtung Insufflatoren betrieben werden.

**Erfindungsgemäße Weiterentwicklungen**

Eine erfindungsgemäße Weiterentwicklung der Vorrichtung besteht darin, dass die Widerstandsbeiwerte jedes Instrumentes auf dem Instrument selbst abgelegt sind oder über dieses ermittelt werden kann. So ist beispielsweise denkbar, an jedem Instrument einen Transponder anzubringen, der Daten enthält, Durch einen entsprechenden Transceiver an der Pumpe können diese Daten ausgelesen werden. Die Daten des Instrumentes können unmittelbar die Widerstandsbeiwerte enthalten. Alternativ können sie auch Identifikationsdaten sein, mittels derer die Widerstandsbeiwerte abgerufen werden können, z.B. vom Pumpenhersteller über das Internet. Weiterhin alternativ können die Daten auch auf anderen Medien gespeichert sein, z.B. auf Barcodes, die ihrerseits mehrdimensional ausgestaltet sein können, oder auf Magnetstreifen.

**Patentansprüche**

**1.** ) Medizintechnische Vorrichtung zum Einbringen von Fluiden in Köperhöhlen unter Bestimmung und Regelung des Körperinnendruckes bei medizintechnischen Verfahren, enthaltend

eine regelbare Fluidpumpe,
eine Zuführleitung, einen Drucksensor in der Zuführleitung, ein an die Zuführleitung anschließbares medizinisches Instrument,
wobei das Fluid durch mindestens eine zweite Leitung aus der Körperhöhle ausfließen kann,
mindestens einen Mikroprozessor, mindestens einen Speicher mit $\zeta_2$ Werten verschiedener medizinischer Instrumente und mindestens eine Software welche zur Durchführung des Verfahrens zur Bestimmung der Widerstandsbeiwerte $\zeta_1$ und $\zeta_2$ wobei
der durch den Drucksensor gemessene Druck eine Eingangsvariable eines mathematischen Schätzsystems ist, welches mathematisch einen Zustandsraum beschreibt, welcher den tatsächlichen Druck in der Körperhöhle abschätzt und mittels dieses Schätzwertes die Leistung der Pumpe regelt, wobei die zur Schätzung des Druckes nötigen Widerstandsbeiwerte $\zeta_1$ und $\zeta_2$ des medizinischen Instrumentes dadurch bestimmt werden, dass für beim Anfahren der Pumpe für eine bestimmte Zeit der Druckverlauf ausgewertet wird,
wobei der Strömungswiderstand $\zeta_1$ als Funktion der messbaren Drehzahl, des messbaren Strömungsdruckes $p_1$, dem nicht messbaren Ruhedruck $p_2$ im Körper sowie einem vorher festgelegten Wert für den Strömungswiderstand $\zeta_2$ , gemäß nachfolgender Gleichung

$$\varsigma_1 = \frac{\Delta p - \varsigma_2 \cdot n_1}{{n_1}^2} = \frac{(p_1 - p_2) - \varsigma_2 \cdot n_1}{{n_1}^2}$$

unter Berücksichtigung des Verlustterms $p_1 = \Delta p + p_2$ für $n_1 > 0$ bestimmt wird,
**dadurch gekennzeichnet, dass**
der Strömungswiderstand $\zeta_2$ innerhalb bestimmter Drehzahlbereiche als konstant angenommen und durch eine kurze, konstante Drehzahlförderung anhand des Druckanstieges aus dem Speicher selektiert wird, hieraus eine Kennlinie ermittelt und die Kennlinie in der Speichereinheit der Pumpe abgespeichert wird.

**2.** ) Medizintechnische Vorrichtung gemäß Anspruch 1, **dadurch gekennzeichnet, dass** die Bestimmung der Widerstandsbeiwerte ζ1 und ζ2 durch zweimaliges Anfahren der Pumpe erfolgt.

**3.** ) Medizintechnische Vorrichtung gemäß Anspruch 1oder 2, **dadurch gekennzeichnet, dass** die Bestimmung der Widerstandsbeiwerte ζ1 und ζ2 des medizinischen Instrumentes prä- oder intraoperativ erfolgt.

**4.** ) Medizintechnische Vorrichtung gemäß Anspruch 1, 2 oder 3, **dadurch gekennzeichnet, dass** das mathematische Schätzsystem nach Art eines Kalman-Filters ausgebildet ist.

**5.** ) Medizintechnische Vorrichtung zum Einbringen von Fluiden in Köperhöhlen gemäß Anspruch 1, **dadurch kennzeichnet, dass** es sich bei der Vorrichtung um einen Insufflator handelt.

**6.** ) Medizintechnische Vorrichtung zum Einbringen von Fluiden in Köperhöhlen gemäß Anspruch 1 oder 5, **dadurch kennzeichnet, dass** es sich bei der Vorrichtung um eine Flüssigkeitspumpe für die Arthroskopie, Urologie, Hysteroskopie, Laparoskopie oder für Wirbelsäulenuntersuchungen handelt.

**7.** ) Medizintechnische Vorrichtung zum Einbringen von Fluiden in Köperhöhlen gemäß Anspruch 1 oder 6, **dadurch kennzeichnet, dass** es sich bei der Vorrichtung um eine Flüssigkeitspumpe mit integrierter Förder- und Absaugpumpe handelt.

## Claims

**1.** A medicinal-technical device for introducing fluids into body cavities uder determination and control of the body's internal pressure with medicinal-technical methods, comprising:

a controllable fluid pump,
a supply line, a pressure sensor in the supply line, a medicinal instrument to be connected to the supply line, wherein the fluid can flow out of the body cavity through at least one second line,
at least one microprocessor, at least one memory with $\zeta_2$ values of different medicinal instruments, and at least one software, which is used for carrying-out the method for determining the drag coefficients $\zeta_1$ and $\zeta_2$,
wherein the pressure measured by the pressure sensor is an input variable of a mathematical estimation system that mathematically describes a state space, which estimates the actual pressure in the body cavity and controls the power of the pump using this estimated value,
wherein the drag coefficients $\zeta_1$ and $\zeta_2$ of the medicinal instrument needed for the estimation of the pressure are determined by that during the start-up of the pump, the pressure trend is evaluated for a certain time,
wherein the drag resistance $\zeta_1$ is determined as a function of the measurable speed of rotation, the measurable flow pressure $p_1$, the non-measurable static pressure $p_2$ in the body, and a pre-deter-' mined value for the drag resistance $\zeta_2$ according to following equation:

$$\varsigma_1 = \frac{\Delta p - \varsigma_2 \cdot n_1}{n_1^{\ 2}} = \frac{(p_1 - p_2) - \varsigma_2 \cdot n_1}{n_1^{\ 2}}$$

under consideration of the loss term $p_1 = \Delta p + p_2$ for $n_1 > 0$,
**characterized by** that the drag resistance $\zeta_2$ is considered constant within certain speed ranges and is selected from the memory by a short, constant speed delivery based on the pressure rise,
therefrom a characteristic line is determined, and the characteristic line is stored in the memory unit of the pump.

**2.** The medicinal-technical device of claim 1, **characterized by** that the determination of the drag coefficients $\zeta_1$ and $\zeta_2$ is made by starting-up the pump twice.

**3.** The medicinal-technical device of claim 1 or 2, **characterized by** that the determination of the drag coefficients $\zeta_1$ and $\zeta_2$ of the medicinal instrument is made pre- or intraoperatively.

**4.** The medicinal-technical device of claim 1, 2, or 3, **characterized by** that the mathematical estimation system is configured as a Kalman filter.

**5.** The medicinal-technical device for introducing fluids into body cavities of claim 1, **characterized by** that the device is an insufflator.

**6.** The medicinal-technical device for introducing fluids into body cavities of claim 1 or 5, **characterized by** that the device is a liquid pump for arthroscopy, urology, hysteroscopy, laparoscopy, or for backbone examinations.

**7.** The medicinal-technical device for introducing fluids into body cavities of claim 1 or 6, **characterized by** that the device is a liquid pump with an integrated transfer and suction pump.

## Revendications

**1.** Dispositif techno-médical pour introduire des fluides dans des cavités du corps en déterminant et régulant la pression interne du corps dans des procédés techno-médicaux, comprenant:

une pompe de fluide réglable,
une conduite d'amenée, un capteur de pression dans la conduite d'amenée, un instrument médical raccordable à la conduite d'amenée,
dans lequel le fluide peut s'écouler de la cavité du corps à travers au moins une deuxième conduite, au moins un microprocesseur, au moins une mémoire avec des valeurs $\zeta_2$ d'instruments médicaux différents, et au moins un logiciel, qui est utilisé pour exécuter le procédé pour déterminer les coefficients de traînée $\zeta_1$ et $\zeta_2$,
dans lequel la pression mesurée par le capteur de pression est une variable d'entrée d'un système d'estimation mathématique qui décrit mathématiquement un espace d'état, qui estime la pression actuelle dans la cavité du corps et règle la puissance de la pompe utilisant cette valeur estimée,
dans lequel les coefficients de traînée $\zeta_1$ et $\zeta_2$ de l'instrument médical nécessaires pour l'estimation de la pression sont déterminés par ce que pendant le démarrage de la pompe, l'évolution en pression est évaluée durant un temps déterminé,
dans lequel la résistance de traînée $\zeta_1$ est déterminée comme fonction de la vitesse de rotation mesurable, la pression d'écoulement $p_1$ mesurable,
la pression statique $p_2$ non mesurable dans le corps et une valeur prédéterminée pour la résistance de traînée $\zeta_2$ selon l'équation suivante:

$$\varsigma_1 = \frac{\Delta p - \varsigma_2 \cdot n_1}{n_1^2} = \frac{(p_1 - p_2) - \varsigma_2 \cdot n_1}{n_1^2}$$

considérant le terme de perte $p_1 = \Delta p + p_2$ pour $n_1 > 0$,
**caractérisé en ce que** la résistance de traînée $\zeta_2$ est considérée comme constante au-dedans de certaines plages de vitesse et est choisie à partir de la mémoire par une délivrance à vitesse courte,
constante sur la base de la montée en pression, à partir de cela une caractéristique est déterminée, et la caractéristique est sauvegardée dans l'unité de stockage de la pompe.

**2.** Dispositif techno-médical selon la revendication 1, **caractérisé en ce que** l'évaluation des coefficients de traînée $\zeta_1$ et $\zeta_2$ se fait par démarrage de la pompe répété deux fois.

**3.** Dispositif techno-médical selon la revendication 1 ou 2, **caractérisé en ce que** l'évaluation des coefficients de traînée $\zeta_1$ et $\zeta_2$ de l'instrument médical se fait pré- ou intraopératoirement.

**4.** Dispositif techno-médical selon la revendication 1, 2, ou 3, **caractérisé en ce que** le système d'estimation mathématique est réalisé comme un filtre de Kalman.

**5.** Dispositif techno-médical pour introduire des fluides dans des cavités du corps selon la revendication 1, **caractérisé en ce que** le dispositif est un insufflateur.

**6.** Dispositif techno-médical pour introduire des fluides dans des cavités du corps selon la revendication 1 ou 5, **caractérisé en ce que** le dispositif est une pompe à liquide pour l'arthroscopie, l'urologie, l'hystéroscopie, la laparoscopie ou pour des examens de la colonne vertébrale.

**7.** Dispositif techno-médical pour introduire des fluides dans des cavités du corps selon la revendication 1 ou 6, **caractérisé en ce que** le dispositif est une pompe à liquide avec une pompe de transfert et d'aspiration intégrée.

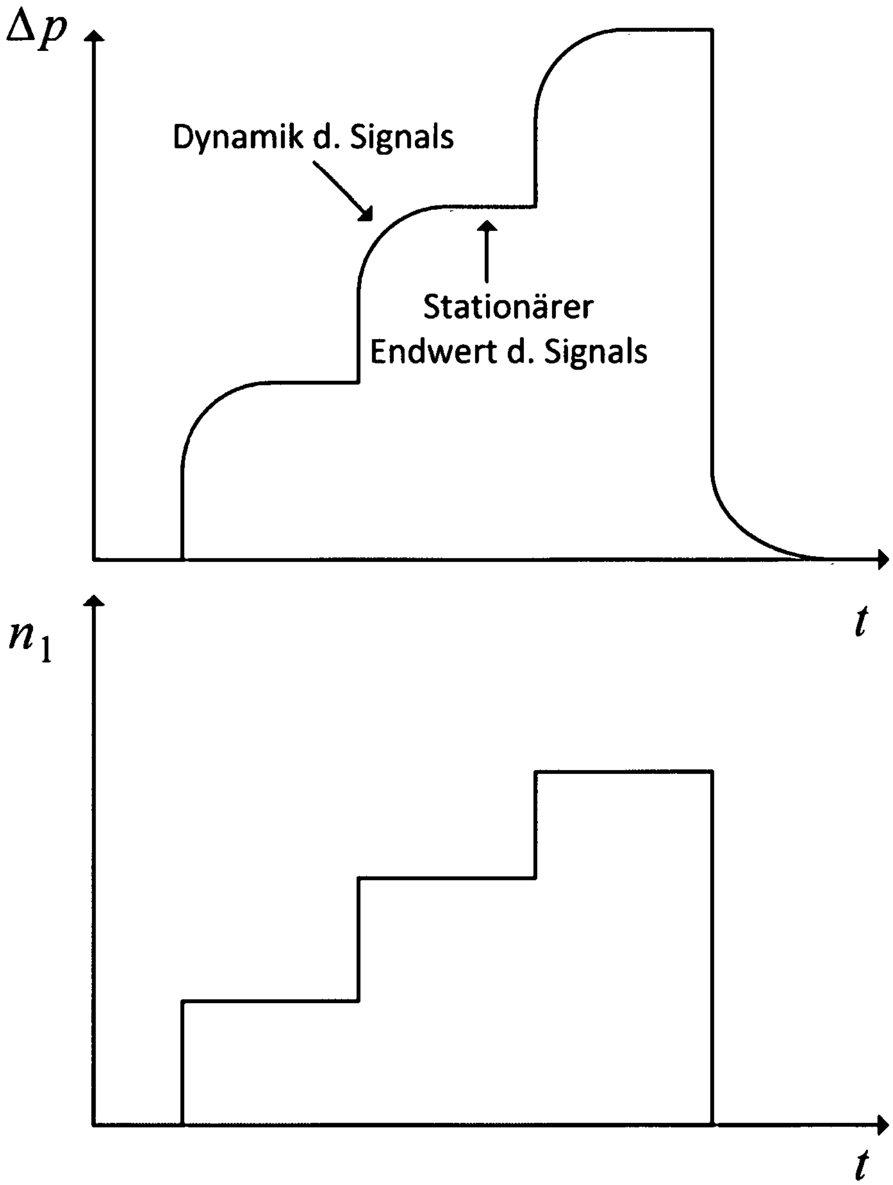

$\Delta p$
Dynamik d. Signals
Stationärer
Endwert d. Signals
t
$n_1$
t

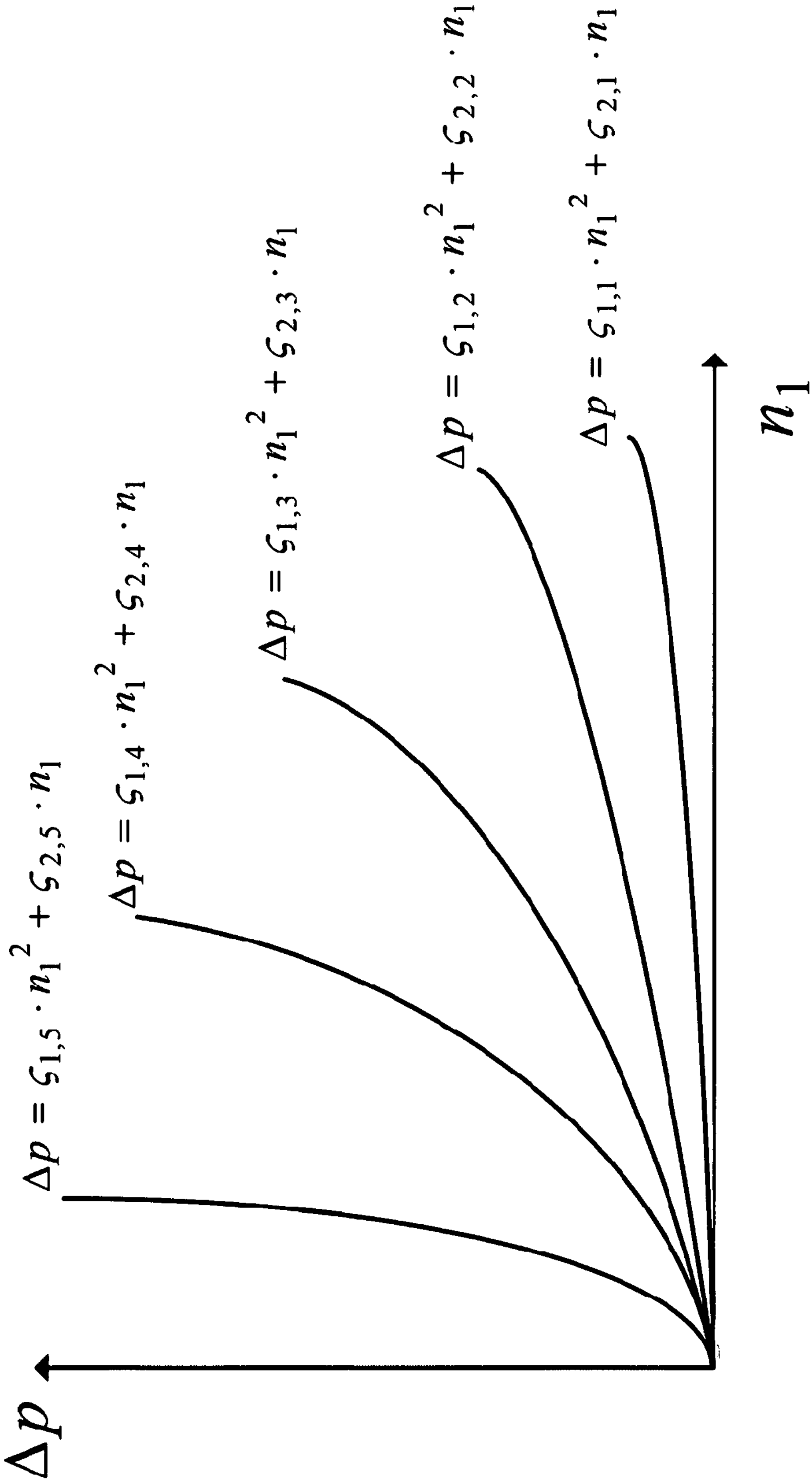

$\Delta p = \varsigma_{1,5} \cdot n_1^{\,2} + \varsigma_{2,5} \cdot n_1$
$\Delta p = \varsigma_{1,4} \cdot n_1^{\,2} + \varsigma_{2,4} \cdot n_1$
$\Delta p = \varsigma_{1,3} \cdot n_1^{\,2} + \varsigma_{2,3} \cdot n_1$
$\Delta p = \varsigma_{1,2} \cdot n_1^{\,2} + \varsigma_{2,2} \cdot n_1$
$\Delta p = \varsigma_{1,1} \cdot n_1^{\,2} + \varsigma_{2,1} \cdot n_1$
$n_1$
$\Delta p$

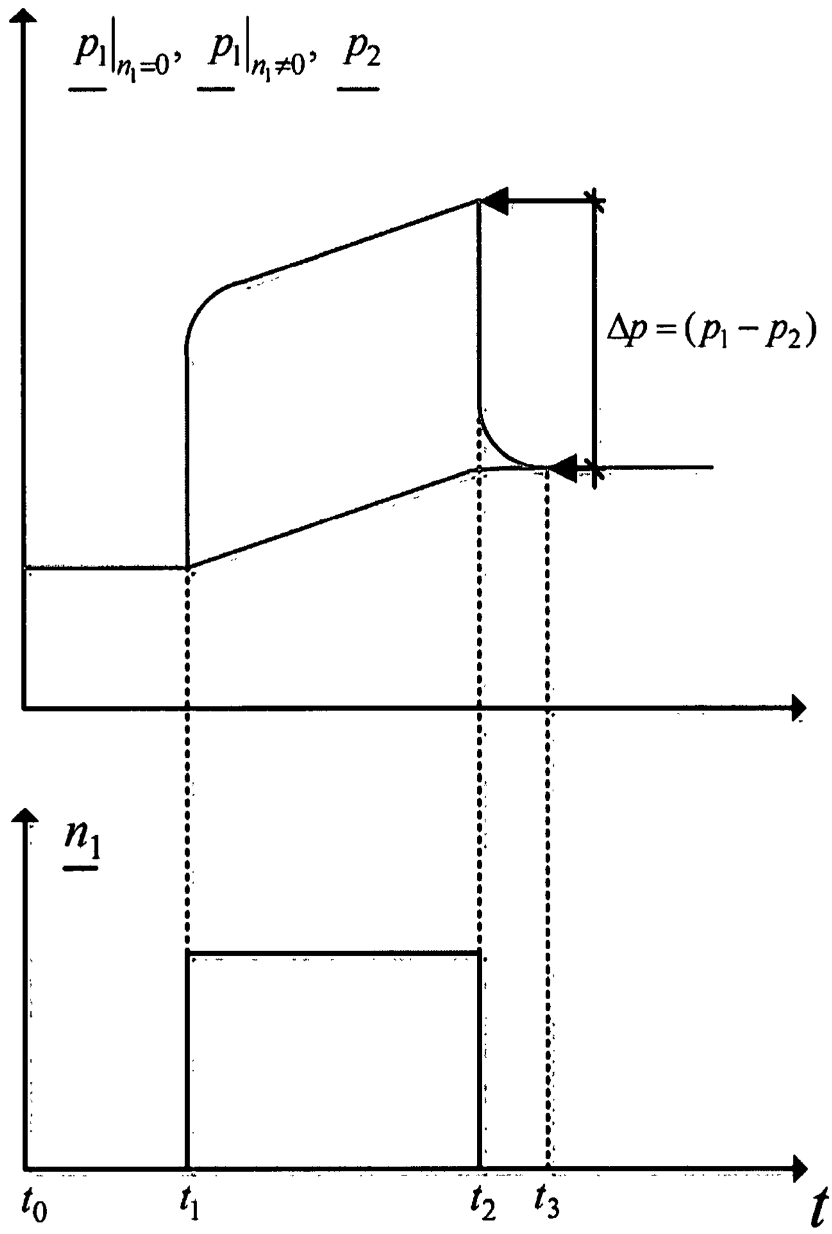
$\underline{p_1}\big|_{n_1=0}$, $\underline{p_1}\big|_{n_1\neq 0}$, $\underline{p_2}$
$\Delta p = (p_1 - p_2)$
$\underline{n_1}$
$t_0$
$t_1$
$t_2$
$t_3$
$t$

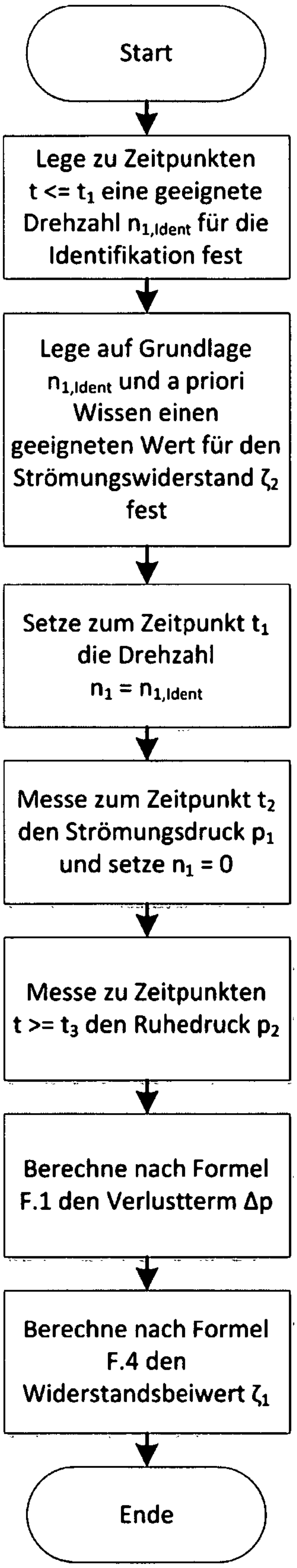
Start
Lege zu Zeitpunkten $t <= t_1$ eine geeignete Drehzahl $n_{1,Ident}$ für die Identifikation fest
Lege auf Grundlage $n_{1,Ident}$ und a priori Wissen einen geeigneten Wert für den Strömungswiderstand $\zeta_2$ fest
Setze zum Zeitpunkt $t_1$ die Drehzahl $n_1 = n_{1,Ident}$
Messe zum Zeitpunkt $t_2$ den Strömungsdruck $p_1$ und setze $n_1 = 0$
Messe zu Zeitpunkten $t >= t_3$ den Ruhedruck $p_2$
Berechne nach Formel F.1 den Verlustterm $\Delta p$
Berechne nach Formel F.4 den Widerstandsbeiwert $\zeta_1$
Ende

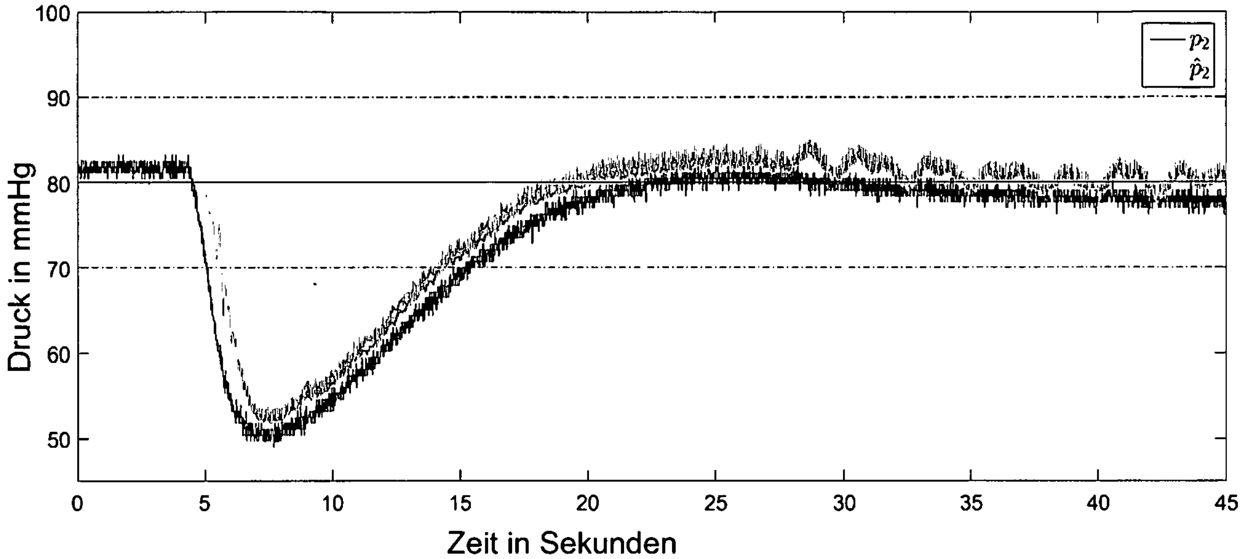

100
90
80
70
60
50
Druck in mmHg
0
5
10
15
20
25
30
35
40
45
Zeit in Sekunden
$p_2$
$\hat{p}_2$


Figur 6 /6:

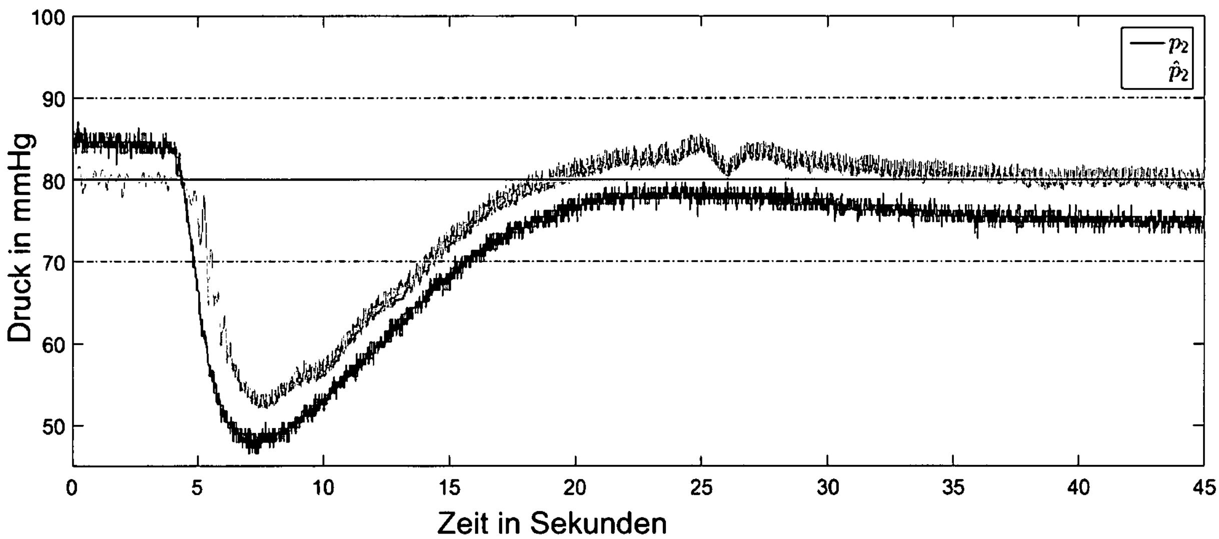

100
90
80
70
60
50
Druck in mmHg
0
5
10
15
20
25
30
35
40
45
Zeit in Sekunden
$p_2$
$\hat{p}_2$

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2015144120 A
- US 20070078370 A1
- US 20130267779 A1